# FASCICULE DE BREVET EUROPEEN

(11) **EP 2 269 592 B1**
(45) Date de publication et mention de la délivrance du brevet: **07.08.2013**
(21) Numéro de dépôt: 09306200.8
(22) Date de dépôt: 09.12.2009
(51) Int. Cl.: A61K 47/44, A61K 9/48, A61K 31/57

(54) **Composition pharmaceutique à base de progestérone micronisée et ses utilisations**
Pharmazeutische Zusammensetzung von mikronisiertem Progesteron und Verwendung derselben
Pharmaceutical composition of micronised progesterone and its use

(30) Priorité: 29.06.2009 FR 0954420
(43) Date de publication de la demande: 05.01.2011
(73) Titulaire: Effik, 92360 Meudon-la-Forêt (FR)
(72) Inventeur: GICQUEL, Jennifer, 78630, ORGEVAL (FR); TERRACOL, Didier, 91370, VERRIERES LE BUISSON (FR); SAMOYEAU, Roland, 92160, ANTONY (FR)
(74) Mandataire: Regimbeau

(56) Documents cités:
- WO-A1-89/02742
- WO-A1-89/05979
- WO-A1-91/18613
- WO-A1-03/041720
- WO-A2-2006/128057
- US-A- 4 900 734
- US-A- 4 963 540

## Description

La présente invention concerne une composition pharmaceutique contenant de la progestérone micronisée et une huile de carthame oléique dite huile de carthame de type II. Elle concerne également des médicaments comprenant ladite composition pharmaceutique.

L'invention concerne en outre les utilisations de cette composition pharmaceutique.

La progestérone est une hormone qui est synthétisée, chez la femme, essentiellement par l'ovaire lors de la phase lutéale, plus précisément par les cellules du corps jaune et, à un moindre degré, par les glandes surrénales et le placenta au cours de la deuxième partie de la grossesse. La concentration plasmatique de progestérone varie pendant le cycle menstruel.

La progestérone est secrétée en plus forte quantité à partir du 14e jour du cycle par les cellules de la granulosa du corps jaune. Elle permet le maintien et la densification de la muqueuse utérine, le développement de la vascularisation de l'endomètre, et l'apparition de glandes utérines responsables de l'aspect dentelé de la paroi utérine.

C'est la progestérone qui permet le maintien et la densification de la muqueuse utérine jusqu'à implantation de l'oeuf dans l'utérus. En cours de grossesse, elle prépare la glande mammaire à la lactation et joue le rôle de relaxant naturel pour éviter les contractions utérines. S'il n'y a pas fécondation, le taux de progestérone revient à la normale.

L'insuffisance en progestérone, dite insuffisance lutéale, peut être responsable de nombreuses pathologies, notamment de fausses couches successives, de troubles du cycle, et du syndrome prémenstruel.

La supplémentation par de la progestérone permet de traiter les insuffisances lutéales, mais son administration par voie orale a souffert de certaines limitations car la progestérone est faiblement absorbée par l'intestin et de plus présente une demi-vie plasmatique relativement brève. En fait, les voies d'administration vaginale, rectale et intramusculaire semblent plus adéquates pour maintenir pendant plusieurs heures consécutives une concentration de progestérone au niveau physiologique de la phase lutéale.

Il est connu dans la demande de brevet FR 76 36007 une formulation contenant de la progestérone micronisée en suspension huileuse, ce qui a permis d'augmenter la biodisponibilité de la progestérone par voie orale. Cette formulation est vendue en France sous la marque UTROGESTAN®.

Cependant, l'huile utilisée est l'huile d'arachide, qui, on le sait, présente des propriétés allergéniques et des réactions d'hypersensibilité importantes. Le brevet US 4,900,734 décrit des compositions contenant de l'huile de carthame de type I, de tournesol, de maïs, de graine de lin, ou des mélanges de ces huiles, de la progestérone micronisée et un oestradiol pour traiter les symptômes de la ménopause.

Le brevet WO 03/041720 a remplacé l'huile d'arachide par d'autres huiles telles que l'huile de tournesol, d'olive, de sésame, de colza ou d'amande qui permettent d'écarter les risques de réactions allergiques tout en conservant l'ensemble des caractéristiques physico-chimiques de la formulation d'UTROGESTAN®. Cependant, cette formulation contient toujours une certaine proportion de lécithine de soja, également connue pour son pouvoir allergénique important. Ce lipide est en effet susceptible d'induire une réponse immunitaire pouvant avoir de graves conséquences avec un risque de survenue de réactions d'hypersensibilité (choc anaphylactique, urticaire). Ce risque a amené les autorités de Santé Publique à qualifier la lécithine de soja d'excipient « à effet notoire » dans le Résumé des Caractéristiques du Produit de UTROGESTAN®.

Il subsiste donc un besoin d'une formulation de progestérone micronisée ne présentant pas ces inconvénients en termes d'allergénicité.

La demanderesse a ainsi découvert qu'en utilisant l'huile de carthame de type II, il était possible de formuler une composition dépourvue de lécithine de soja tout en conservant les qualités de dispersion de la progestérone micronisée dans le médicament.

Un objet de l'invention est donc une composition pharmaceutique comprenant de la progestérone micronisée dans de l'huile de carthame de type II.

Un autre objet de l'invention est l'utilisation de progestérone micronisée dans de l'huile de carthame de type II pour le traitement d'une condition physiologique liée à l'insuffisance de la sécrétion de la progestérone.

D'autres objets apparaîtront à la lecture de la description et des exemples qui suivent.

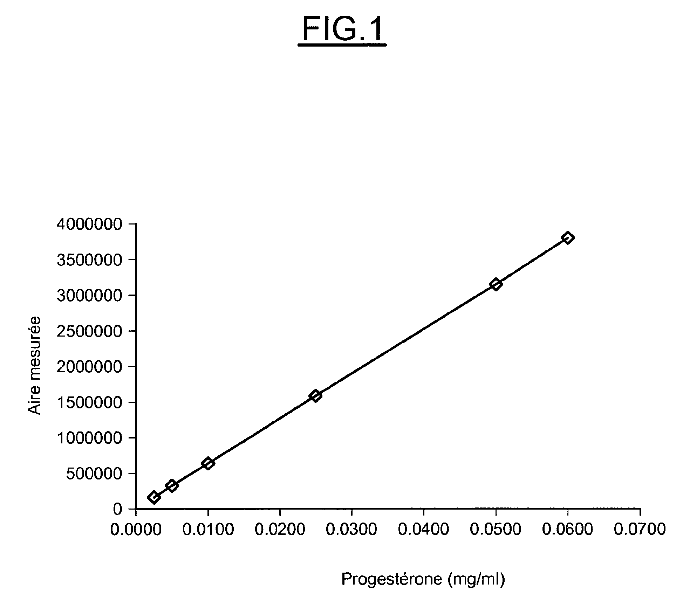

La figure 1 montre une linéarité entre l'aire du pic mesurée et la quantité de progestérone dissoute dans un milieu huileux. La limite de détection (LOD) de la progestérone est de 0.38 microgrammes/mL et la limite de quantification (LOQ) de la progestérone est de 1.16 microgrammes/mL.

La demanderesse a recherché, parmi toutes les huiles disponibles, celle qui ne présentait pas de risque allergénique et qui offrait le meilleur profil nutritionnel. La demanderesse a pu observer que l'huile de carthame de type II s'est révélée apporter en plus un bénéfice technique considérable dans la mesure où elle permet de s'affranchir, dans la composition, de la lécithine de soja potentiellement allergénique, sans altérer la qualité de la mise la suspension de la progestérone micronisée dans l'huile lors de la préparation.

Le choix de cette huile de carthame de type II permet donc de mieux maîtriser le taux de sédimentation de la composition progestérone dans l'huile. Dans la composition pharmaceutique selon l'invention, la progestérone micronisée se trouve ainsi en suspension dans l'huile de carthame de type II.

L'huile de carthame de type II est extraite des graines de carthame, une plante originaire d'Égypte. On la retrouve également à l'état sauvage en Chine, Inde, Japon, Australie, Iran et dans toute l'Amérique. Il s'agit d'une huile pauvre en graisses saturées. L'huile de carthame de type II est de plus extrêmement intéressante en nutrition pour sa richesse en acides gras essentiels insaturés, incluant les acides linoléique et oléique. De plus c'est une huile très pauvre en acides gras saturés.

La composition moyenne en acides gras de l'huile de carthame de type II est ainsi approximativement de 65 à 85 % d'acide oléique et de 5 à 25 % d'acide linoléique. Cette huile est ainsi une source importante d'acides gras monoinsaturés, particulièrement l'acide oléique. Cet acide gras est nécessaire pour le rétablissement de la balance Omega-6/Omega-3 en raison de sa neutralité vis-à-vis des enzymes du métabolisme des acides gras essentiels.

Dans le contexte de la présente invention, l'huile de carthame de type II utilisée peut être raffinée ou non. Une huile raffinée est une huile qui est obtenue en partant de l'huile brute et qui a subi un ensemble d'opérations de raffinage. L'huile raffinée est une huile purifiée présentant un très faible taux d'impuretés et exempte notamment de protéines potentiellement allergisantes telles que le gluten.

Il est possible d'utiliser l'huile de carthame de type II de la société Olisud, Aix en Provence, France (huile de carthame oléique raffinée CAS 8001-23-8).

Dans le contexte de la présente invention, on entend par progestérone micronisée une progestérone dont au moins 99% des particules ont une granulométrie inférieure à 60 µm, cette granulométrie étant mesurée par granulomètre laser de type Malvern selon un procédé connu (Delgrove R., et J.M. Hochart, AVH Association, 8th symposium Reims, March 2001).

De préférence, le rapport progestérone/huile est compris entre 0,15/1 et 3/1, de préférence entre 0,25/1 et 2/1, préférentiellement entre 0,40/1 et 1/1, et plus préférentiellement encore est de 0,67/1.

On peut préparer une composition pharmaceutique selon l'invention en ajoutant progressivement, sous agitation à environ 1000 tours/min, la progestérone micronisée à l'huile de carthame de type II, puis en maintenant l'agitation à la même vitesse pendant approximativement une heure afin d'obtenir une suspension homogène.

La composition pharmaceutique selon l'invention peut également comprendre un oestrogène ou un de ses dérivés de type ester, de préférence sélectionné dans le groupe constitué par le 17-β estradiol, l'estrone, le 17-α éthinyl-estradiol, le valérate d'estradiol, ou les phyto- oestrogènes, et plus préférentiellement encore le 17-β estradiol.

La composition pharmaceutique selon l'invention peut contenir un agent de suspension comme l'aérosil (Aérosil R972 Pharma, France) à une quantité de 0,1 à 1% de la composition selon l'invention, préférentiellement de 0,4 à 0,6 % et encore plus préférentiellement à un pourcentage de 0,5% de la composition selon l'invention.

La composition pharmaceutique selon l'invention peut se présenter sous la forme d'une capsule molle. La capsule molle contenant la composition selon l'invention peut également comprendre des agents liants, des agents désintégrants, des agents diluants et/ou des agents lubrifiants.

Selon un mode avantageux de réalisation de la composition pharmaceutique selon l'invention, la capsule comprend de la gélatine ou équivalent.

Lorsque la composition pharmaceutique selon l'invention est intégrée dans une spécialité pharmaceutique, chaque unité posologique comprend avantageusement entre 2 mg et 600 mg de progestérone micronisée, et plus préférentiellement encore entre 100 mg et 400 mg.

La composition pharmaceutique selon l'invention peut être administrée par voie orale ou par voie vaginale selon les indications thérapeutiques.

L'administration par voie vaginale représente également une alternative à la voie orale en cas d'effets secondaires dus à la progestérone (somnolence après absorption par voie orale) ou de contre-indication à la voie orale (hépatopathie).

La progestérone micronisée dans de l'huile de carthame de type II est ainsi utilisée dans le traitement d'une condition physiologique liée à l'insuffisance de la sécrétion de la progestérone

L'invention concerne également l'utilisation de la progestérone micronisée dans de l'huile de carthame de type II pour la préparation d'un médicament pour le traitement d'une condition physiologique liée à l'insuffisance de la sécrétion de la progestérone.

On peut citer, comme exemples de telles conditions physiologiques : l'insuffisance lutéale, l'irrégularité menstruelle, les syndromes prémenstruels, les mastodynies, les mastopathies bénignes, la préménopause, la stérilité par insuffisance lutéale, les troubles de la ménopause, la contraception locale, la prévention d'avortements habituels en cas d'insuffisance lutéale, les menaces d'accouchement prématuré, la prévention de l'ostéoporose, les hyperplasies et les cancers de l'endomètre, et l'épilepsie.

L'invention concerne également l'utilisation de la progestérone micronisée et de l'huile de carthame de type II associées à un oestrogène, pour la préparation d'un médicament pour le traitement d'une condition physiologique liée à l'insuffisance de la sécrétion de la progestérone. L'oestrogène ou un de ses dérivés de type ester est de préférence sélectionné dans le groupe constitué par le 17-β estradiol, l'estrone, le 17-α éthinyl-estradiol, le valérate d'estradiol, ou les phyto-oestrogènes, et plus préférentiellement encore le 17-β estradiol.

Les exemples suivants illustrent l'invention sans la limiter aucunement.

### Exemple 1 : mesure de la sédimentation par centrifugation de progestérone dans la composition selon l'invention.

Différents mélanges huile/progestérone micronisée ont été réalisés en introduisant dans un bécher de 600 millilitres, x millilitres d'huile à tester, sous agitation à 1000 tours/min avec une pale papillon tout en ajoutant progressivement la progestérone micronisée. Les mélanges sont ensuite agités pendant une heure à 1000 tours/min avec une pale papillon. Les huiles utilisées proviennent d'Olisud, Aix en Provence, France (huile d'arachide raffinée lot L805160, huile d'onagre raffinée lot L710020, huile de pépin de raisin raffinée lot L804231, huile de carthame oléique, dite carthame de type II raffinée lot L709271. La progestérone micronisée provient de Shenzhou, Zejiang, Chine, lot Prob-080304).

Le test de sédimentation est ensuite réalisé par centrifugation des mélanges à 4000 tours /min pendant des temps de 10, 15 et 20 minutes.

Le volume moyen de surnageant par centrifugation est ensuite prélevé par aspiration, mesuré et pesé à différents temps après centrifugation. Le poids est converti en volume en divisant celui-ci par la masse volumique de l'huile considérée et la pente moyenne de régression est calculée en millilitres par minutes.

**Tableau 1 : mesure de la sédimentation de la progestérone selon les huiles utilisées**

| Huile | | Carthame II | Tournesol | Olive | Arachide | Colza | Carthame 1 |
|---|---|---|---|---|---|---|---|
| Essai 1 : | | | | | | | |
| volumes à | | | | | | | |
| | - 10 min (ml) | 1,21 | 1,62 | 1,45 | 1,65 | 1,72 | 1,83 |
| | - 15 min (ml) | 2,22 | 2,52 | 2,73 | 2,97 | 3,34 | 3,42 |
| | - 20 min (ml) | 3,29 | 4,00 | 4,33 | 4,51 | 5,09 | 5,12 |
| | | | | | | | |
| Pente (ml/min) | | 0,209 | 0,238 | 0,288 | 0,286 | 0,337 | 0,329 |
| | | | | | | | |
| Essai 1 : | | | | | | | |
| volumes à | | | | | | | |
| | - 10 min (ml) | 1,13 | 1,58 | 1,40 | 1,62 | 1,70 | 1,75 |
| | - 15 min (ml) | 2,19 | 2,49 | 2,70 | 2,86 | 3,35 | 3,33 |
| | - 20 min (ml) | 3,39 | 4,13 | 4,25 | 4,39 | 4,69 | 5,03 |
| | | | | | | | |
| Pente (ml/min) | | 0,226 | 0,254 | 0,285 | 0,277 | 0,300 | 0,328 |
| | | | | | | | |
| | V. moyen à 20 min (ml) | 3,34 | 4,07 | 4,29 | 4,45 | 4,89 | 5,08 |
| | Pente moyenne (ml/min) | 0,218 | 0,246 | 0,286 | 0,281 | 0,318 | 0,329 |

Le tableau 1 montre nettement que la sédimentation de progestérone la moins importante est obtenue avec l'huile de carthame de type II par comparaison avec de l'huile d'arachide, de tournesol, de colza, d'olive ou de carthame de type I.

### Exemple 2 : mesure de la viscosité de la composition selon l'invention

Différents mélanges huile/progestérone micronisée ont été réalisés en introduisant dans un bécher de 600 millilitres, x millilitres d'huile à tester, sous agitation à 1000 tours/min avec une pale papillon tout en ajoutant progressivement la progestérone micronisée. Les mélanges sont ensuite agités pendant une heure à 1000 tours/min avec une pale papillon. Les huiles utilisées proviennent d'Olisud, Aix en Provence, France (huile d'arachide raffinée lot L805160, huile d'onagre raffinée lot L710020, huile de pépin de raisin raffinée lot L804231, huile de carthame oléique, dite carthame de type II raffinée lot L709271. La progestérone micronisée provient de Shenzhou, Zejiang, Chine, lot Prob-080304).

Pour ces mesures, le volume de l'échantillon testé est de 30 grammes ± 0,5g à une température de 22°C pour chaque mélange; la viscosité est mesurée après mélange et après environ deux heures de repos.

**Tableau 2 : mesure de la viscosité des huiles utilisées**

| Huile | Colza | Carthame I | Tournesol | Olive | Arachide | Carthame II |
|---|---|---|---|---|---|---|
| T0 | 5590 | 5740 | 6210 | 6210 | 6850 | 6550 |
| T2h | 5110 | 5780 | 6620 | 6750 | 7050 | 7350 |

Le tableau 2 montre ainsi que les viscosités sont sensiblement équivalentes suivant les huiles utilisées, la viscosité de l'huile de carthame de type_II étant très proche de celle de l'huile d'arachide.

### Exemple 3 : comparaison de l'huile selon l'invention avec une formulation semblable à celle de l'Utrogestan®.

Les paramètres physico-chimiques de l'huile de carthame de type II ont été testés en comparaison avec une formulation contenant de l'huile d'arachide et 0,4% de lécithine de soja, selon le protocole décrit précédemment.

Les résultats sont exprimés dans le tableau 3 suivant.

**Tableau 3 : comparaison des huiles en tenant compte de la présence de lécithine de soja**

| | Viscosité à T 2h (mPa.s) | Sédimentation |
|---|---|---|
| Huile arachide + lécithine de soja 0,4% | 6610 | 3,61 |
| Huile de carthame de type II | 7350 | 3,34 |

On voit ainsi que la modification de l'huile dans les formulations de progestérone micronisée n'affecte pas les propriétés physico-chimiques de la composition selon l'invention.

### Exemple 4 : mesures des teneurs en progestérone solubilisée dans la composition selon l'invention

Différents mélanges huile/progestérone micronisée ont été réalisés en introduisant dans un bécher de 600 millilitres, x millilitres d'huile à tester, sous agitation à 1000 tours/min avec une pale papillon tout en ajoutant progressivement la progestérone micronisée. Les mélanges sont ensuite agités pendant une heure à 1000 tours/min avec une pale papillon. Les huiles utilisées proviennent d'Olisud, Aix en Provence, France (huile d'arachide raffinée lot L805160, huile d'onagre raffinée lot L710020, huile de pépin de raisin raffinée lot L804231, huile de carthame oléique, dite carthame de type II raffinée lot L709271. La progestérone micronisée provient de Shenzhou, Zejiang, Chine, lot Prob-080304).

Le test de solubilisation est réalisé par centrifugation des mélanges à 4000 tours /min pendant des temps de 10, 15 et 20 minutes.

Le dosage de la progestérone solubilisée est ensuite réalisé sur une prise d'essai du surnageant total collecté après centrifugation sur les échantillons de l'exemple 1.

**Tableau 4 : mesure de la teneur en progestérone solubilisée**

| Formulation | Teneur en progestérone dans l'huile en % m/m |
|---|---|
| Témoin huile arachide raffinée + lécithine de soja | 1,86 |
| Huile d'arachide raffinée | 1,84 |
| Huile de carthame type II raffinée | 1,90/1,82* |
| Huile d'onagre raffinée | 2,15 |
| Huile de pépins de raisin raffinée | 2,14 |
| Huile de tournesol | 2,13 |

| | |
|---|---|
| *teneur après 43h d'agitation | |

Le tableau 4 montre que le mélange comprenant de l'huile de carthame de type II a une teneur en progestérone solubilisée semblable à celle des mélanges comprenant de huile d'arachide raffinée avec ou sans lécithine de soja.

### Exemple 5 : Compositions pharmaceutiques selon l'invention

| Formules de compositions | Viscosité à T=0 (mPa.s) | Sédimentation (ml/mm) |
|---|---|---|
| Progestérone 100 mg | 6710 | 0,255 |
| Huile carthame type II oléique 150 mg | | |
| Progestérone 100 mg | 5980 | 0,257 |
| Huile carthame type II oléique 149,4 mg | | |
| Aerosil 0,625 mg | | |
| Témoin 1 | 7110 | 0,285 |
| Progestérone 100 mg | | |
| Huile arachide 150 mg | | |
| Témoin 2 | 6650 | 0,257 |
| Progestérone 100 mg | | |
| Huile arachide 149 mg | | |
| Lécithine soja 1mg | | |

## Revendications

1. Composition pharmaceutique comprenant de la progestérone micronisée et de l'huile de carthame de type II, **caractérisée en ce que** dans ladite composition au moins la progestérone se trouve en suspension dans l'huile de carthame de type II.

2. Composition pharmaceutique selon la revendication 1, dans laquelle le rapport progestérone / huile est compris entre 0, 15/ 1 et 3/1, de préférence entre 0,25/1 et 2/1, préférentiellement entre 0,40/1 et 1/1, et plus préférentiellement encore est de 0,67/1.

3. Composition pharmaceutique selon l'une quelconque des revendications 1 ou 2, comprenant un oestrogène ou un dérivé de type ester, de préférence sélectionné dans le groupe constitué par le 17-β estradiol, l'estrone, le 17-α éthinyl-estradiol, le valérate d'estradiol, ou les phyto-oestrogènes, et plus préférentiellement encore est le 17-β estradiol.

4. Composition pharmaceutique selon l'une quelconque des revendications 1 à 3, comprenant un agent de suspension.

5. Composition selon la revendication 4, dans laquelle l'agent de suspension est l'Aérosil.

6. Composition pharmaceutique selon l'une quelconque des revendications 1 à 5, se présentant sous la forme d'une capsule molle.

7. Spécialité pharmaceutique comprenant la composition selon l'une quelconque des revendications 1 à 6, chaque unité posologique de ladite spécialité comprenant entre 2 mg et 600 mg de progestérone micronisée, et plus préférentiellement entre 100 mg et 400 mg.

8. Composition selon l'une quelconque des revendications 1 à 6 ou spécialité selon la revendication 7 pour son utilisation dans le traitement d'une condition physiologique liée à l'insuffisance de la sécrétion de la progestérone.

9. Utilisation de la composition selon l'une quelconque des revendications 1 à 6 ou de la spécialité selon la revendication 7 pour la préparation d'un médicament destiné au traitement d'une condition physiologique liée à l'insuffisance de la sécrétion de la progestérone.

10. Utilisation selon la revendication 9, dans laquelle le médicament contient également un oestrogène ou un dérivé de type ester, de préférence sélectionné dans le groupe constitué par le 17-β estradiol, l'estrone, le 17-α éthinyl-estradiol, le valérate d'estradiol, ou les phyto-oestrogènes, et plus préférentiellement encore est le 17-β estradiol.

11. Utilisation selon l'une quelconque des revendications 9 ou 10, dans laquelle le médicament contient également un agent de suspension, comme l'Aérosil.

## Patentansprüche

1. Pharmazeutische Zusammensetzung, umfassend mikronisiertes Progesteron und Saflor-Distelöl vom Typ II, **dadurch gekennzeichnet, dass** in der Zusammensetzung zumindest das Progesteron suspendiert in dem Saflor-Distelöl vom Typ II vorliegt.

2. Pharmazeutische Zusammensetzung nach Anspruch 1, wobei das Verhältnis Progesteron/Öl zwischen 0,15/1 und 3/1, bevorzugt zwischen 0,25/1 und 2/1, weiter bevorzugt zwischen 0,40/1 und 1/1, und noch weiter bevorzugt bei 0,67/1 liegt.

3. Pharmazeutische Zusammensetzung nach einem der Ansprüche 1 oder 2, umfassend ein Östrogen oder ein Derivat vom Estertyp, welches bevorzugt ausgewählt ist aus der Gruppe bestehend aus 17-β-Estradiol, Estron, 17-α-Ethinyl-Estradiol, Estradiol-Valerat oder Phyto-Östrogenen, und welches noch weiter bevorzugt 17-β-Estradiol ist.

4. Pharmazeutische Zusammensetzung nach einem der Ansprüche 1 bis 3, umfassend ein Suspensionsmittel.

5. Zusammensetzung nach Anspruch 4, wobei das Suspensionsmittel Aerosil ist.

6. Pharmazeutische Zusammensetzung nach einem der Ansprüche 1 bis 5, die in Form einer Weichkapsel vorliegt.

7. Pharmazeutische Spezialität, umfassend die Zusammensetzung nach einem der Ansprüche 1 bis 6, wobei jede posologische Einheit der Spezialität zwischen 2 mg und 600 mg mikronisiertes Progesteron umfasst, weiter bevorzugt zwischen 100 mg und 400 mg.

8. Zusammensetzung nach einem der Ansprüche 1 bis 6 oder Spezialität nach Anspruch 7 zur Verwendung bei der Behandlung eines physiologischen Zustandes, der mit einer Insuffizienz der Progesteron-Sekretion zusammenhängt.

9. Verwendung der Zusammensetzung nach einem der Ansprüche 1 bis 6 oder der Spezialität nach Anspruch 7 für die Herstellung eines Medikaments, welches zur Behandlung eines physiologischen Zustandes bestimmt ist, der mit einer Insuffizienz der Progesteron-Sekretion zusammenhängt.

10. Verwendung nach Anspruch 9, wobei das Medikament auch ein Östrogen oder ein Derivat vom Estertyp umfasst, welcher bevorzugt ausgewählt ist aus der Gruppe bestehend aus 17-β-Estradiol, Estron, 17-α-Ethinyl-Estradiol, Estradiol-Valerat oder Phyto-Östrogenen, und welches noch weiter bevorzugt 17-β-Estradiol ist.

11. Verwendung nach einem der Ansprüche 9 oder 10, wobei das Medikament auch ein Suspensionmittel wie Aerosil umfasst.

## Claims

1. Pharmaceutical composition comprising micronised progesterone and type II safflower oil, **characterised in that**, in said composition, at least the progesterone is in suspension in the type II safflower oil.

2. Pharmaceutical composition according to claim 1, wherein the progesterone to oil ratio is between 0.15:1 and 3:1, preferably between 0.25:1 and 2:1, preferentially between 0.40:1 and 1:1, and more preferentially is 0.67:1.

3. Pharmaceutical composition according to any of claims 1 or 2, comprising an oestrogen or an ester type derivative, preferably selected in the group consisting of 17-β oestradiol, oestrone, 17-α ethinyl-oestradiol, oestradiol valerate or plant oestrogens, and more preferentially is 17-β oestradiol.

4. Pharmaceutical composition according to any of claims 1 to 3, comprising a suspension agent.

5. Composition according to claim 4, wherein the suspension agent is Aerosil.

6. Pharmaceutical composition according to any of claims 1 to 5, in soft capsule form.

7. Pharmaceutical product comprising the composition according to any of claims 1 to 6, each dosage unit of said product comprising between 2 mg and 600 mg of micronised progesterone and more preferentially between 100 mg and 400 mg.

8. Composition according to any of claims 1 to 6 or product according to claim 7 for the use thereof in the treatment of a physiological condition associated with progesterone secretion deficiency.

9. Use of the composition according to any of claims 1 to 6 or of the product according to claim 7 for preparing a medicinal product for treating a physiological condition associated with progesterone secretion deficiency.

10. Use according to claim 9, wherein the medicinal product also contains an oestrogen or an ester type derivative, preferably selected in the group consisting of 17-β oestradiol, oestrone, 17-α ethinyl-oestradiol, oestradiol valerate or plant oestrogens, and more preferentially is 17-β oestradiol.

11. Use according to any of claims 9 or 10, wherein the medicinal product also contains a suspension agent, such as Aerosil.
